# EUROPEAN PATENT SPECIFICATION

(11) **EP 0 538 229 B1**
(45) Date of publication and mention of the grant of the patent: **10.07.1996**
(21) Application number: 92870158.0
(22) Date of filing: 29.09.1992
(51) Int. Cl.: C07C 6/12, C07C 15/04, C07C 15/08

(54) **A start-up process for improved selectivity in toluene disproportionation**
Anlaufprozess zur verbesserten Selektivität der Toluol-Disproportionierung
Procédé de démarrage pour améliorer la sélectivité de disproportionnement de toluène

(30) Priority: 15.10.1991 US 776937; 15.10.1991 US 776031
(43) Date of publication of application: 21.04.1993
(73) Proprietor: FINA TECHNOLOGY, INC., Dallas, Texas 75206 (US)
(72) Inventor: Shamshoum, Edwar S., Houston, Texas 77062 (US); Ghosh, Ashim K., Houston, Texas 77062 (US); Schuler, Thomas R., Galena Park, Texas 77547 (US)
(74) Representative: Leyder, Francis

(56) References cited:
- EP-A- 0 308 096
- US-A- 4 665 258
- US-A- 4 956 511

## Description

### BACKGROUND OF THE INVENTION

### Field of the Invention

This invention relates to a start-up procedure for use in the disproportionation of alkylaromatic feed-streams and, more particularly, in the disproportionation of toluene containing feedstocks employing mordenite catalysts of relatively high aluminum content.

### Description of the Related Art

The disproportionation of toluene involves a well known transalkylation reaction in which toluene is converted to benzene and xylene in accordance with the following reaction: 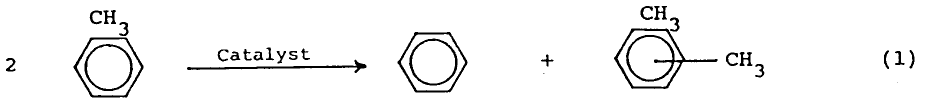
Reaction (1) is mildly exothermic.

Mordenite is one of a number of molecular sieve catalysts useful in the transalkylation of alkylaromatic compounds. As a crystalline aluminosilicate zeolite, mordenite exhibits a network of silicon and aluminum atoms interlinked by oxygen atoms within the crystalline structure. For a general description of mordenite catalysts, reference is made to Kirk-Othmer, Encyclopedia of Chemical Technology, 3rd Edition, 1981, under the heading "Molecular Sieves", Vol. 15, pages 638-643. Mordenite as found in nature or as synthesized to replicate the naturally occurring zeolite, typically exhibits a relatively low silica to alumina mole ratio of about 10 or less. Also known, however, are mordenite catalysts exhibiting a substantially lower alumina content. These aluminum deficient mordenite catalysts exhibit silica to alumina ratios greater than 10, ranging up to about 100, and may be prepared by direct synthesis as disclosed, for example, in U.S. Patent No. 3,436,174 to **Sand** or by acid extraction of a more conventionally prepared mordenite as disclosed in U.S. Patent No. 3,480,539 to **Voorhies**, **et. al**. Both the typical and the aluminum deficient mordenites are known to be useful in the disproportionation of toluene.

Disproportionation of toluene feedstock may be performed at temperatures ranging from about 200°C to about 600°C or above and at pressures ranging from atmospheric to perhaps 100·10⁵ Pa (100 atmospheres) or above. The specific catalyst, however, may impose constraints on reaction temperatures thus affecting catalyst activity and aging character. In general, the prior art suggests the use of relatively high temperatures when employing the high aluminum mordenites (low silica to alumina ratios) and somewhat lower temperatures when employing the low alumina mordenites. Accordingly, where mordenite catalysts exhibiting high silica/alumina ratios have been employed in the transalkylation of alkylaromatics, it has been the practice to operate toward the lower end of the temperature range. U.S. Patent No. 4,665,258 to **Butler, et. al.**, however, discloses disproportionation of a toluene containing feedstock employing an aluminum deficient mordenite catalyst under relatively severe disproportionation conditions; involving a temperature range of 370°-500°C. The mordenite catalysts exhibit silica/alumina mole ratios of at least 30 and, more desirably, within the range of 40-60. The feedstock may be supplied to the reaction zone containing the mordenite catalyst at rates providing relatively high space velocities. The toluene weight hourly space velocity (WHSV) may be greater than 1. Hydrogen is supplied to the reaction zone at a hydrogen/toluene mole ratio within the range of 3-6. The hydrogen pressure may be 34.47 ·10⁵ Pa (500 psi) or more. The toluene feedstock need not be dried before supplying it to the reaction zone and the patent discloses toluene feedstocks exhibiting a water contentin excess of 100 ppm.

**Butler, et al.** also discloses passing a hot preflush gas, nitrogen or hydrogen, to the reaction zone prior to initiating the disproportionation reaction. The preflush gas is heated to a temperature sufficient to substantially dehydrate the catalyst by the time the toluene feed is started. This measure enables the disproportionation process to initially be performed at a somewhat lower temperature and without reduction in toluene conversion. As the disproportionation proceeds, temperature progressively increases to maintain toluene conversion at the desired level, typically about 80 percent of theoretical. U. S. Patent No. 4,723,049 to **Menard et al.** discloses toluene disproportionation carried out over aluminum deficient mordenite of the type disclosed in the aforementioned patent to Butler. In this process, preferably carried out at a reaction zone temperature of 370°-500°C, and more preferably at a temperature of 400°-500°C with an unmodified aluminum deficient mordenite catalyst, the supply of toluene to the reaction zone is interrupted while the supply of hydrogen is continued. Preferably the period of interruption during which hydrogen supply is continued is for at least one day prior to reinstating the supply of toluene feedstock to the reaction zone. This mode of operation is disclosed to enhance the aging quality of the catalyst and show a reduction in reactor zone temperature without a corresponding decrease in toluene conversion.

It is also a common practice to promote an aluminum deficient mordenite catalyst with a catalytically active metallic content. For example, U.S. Patent No. 3,476,821 to **Brandenburg et al.** discloses disproportionation reactions employing mordenite catalysts having silica/alumina ratios within the range of 10-100 and preferably within the range of about 20-60. The mordenites are modified by the inclusion of a sulfided metal selected from the Group VIII metals. The metal may be included in the mordenite by well known ion exchange or impregnation techniques. The especially preferred sulfided Group VIII metals are cobalt and nickel present in a concentration of 0.5-10 weight percent. When compared with nickel oxide, nickel sulfide is said to provide less overactivity as indicated by gas and saturated hydrocarbon yield. Here the desired temperature ranges are said to be from about 204.4-389.9°C (400°-750°F) and preferebly 232.2-337.8°C (450°-640°F). The metal promoters are said to substantially increase activity and catalyst life, as indicated by runs extending over several hours or days.

As noted previously, hydrogen is supplied along with toluene to the reaction zone. While the disproportionation reaction (1) does not involve chemical consumption of hydrogen, the use of a hydrogen cofeed is generally considered to prolong the useful life of the catalyst, as disclosed, for example, in the above mentioned patent to **Brandenburg**. The amount of hydrogen supplied, which is normally measured in terms of the hydrogen/toluene mole ratio, is generally shown in the prior art to increase as temperature increases.

**Bhavikatti et al.,** "Toluene Disproportionation Over Aluminum-Deficient and Metal-Loaded Mordenites. 1. Catalytic Activity and Aging", Ind. Eng. Chem. Prod. Res. Dev. 1981, 20, 102-105, discloses toluene disproportionation at 400°C over mordenite catalysts having silica/alumina mole ratios ranging from 12 to 61 at atmospheric pressure and a space velocity (WHSV) of 1. As the silica/alumina mole ratio is increased, catalyst activity is substantially decreased while aging quality is increased. That is, the aging rates were lower. Based upon short term aging studies, the best silica/alumina mole ratio appeared to be 23. Catalyst decay was also suppressed by loading the mordenites with nickel. Mordenites having a silica/alumina ratio of 12, 16 and 23 were modified by the inclusion of nickel by a procedure involving ion exchanging ammonium mordenite with an aqueous solution of nickel nitrate. After ion exchange, the catalyst was activated under a hydrogen environment for two hours. The best activation temperature for nickel modified mordenite having a silica/alumina ratio of 23 was indicated to be about 550°C. The nickel modified mordenite having a silica/alumina ratio of 12 showed significantly lower activity when compared to the nickel loaded mordenite of a silica/alumina ratio of 23.

U.S. Patent No. 3,562,345 to **Mitsche** discloses the use of molecular sieves such as mordenite catalysts in the disproportionation of toluene. The catalysts are characterized by a silica/alumina mole ratio from about 6 to about 12, pore openings of from about 3 to about 18 angstroms and the incorporation of catalytically active metallic materials in the oxidized or reduced state, particularly Group VIB and Group VIII metals including molybdenum, tungsten, chromium, iron, nickel, cobalt, platinum, palladium, ruthenium, rhodium, osmium, and iridium. **Mitsche** discloses transalkylation at temperatures from about 200°C to about 480°C and gives specific examples of transalkylation of toluene at temperatures of 420°C and 450°C.

U.S. Patent No. 3,677,973 to **Mitsche et. al.**, discloses the use of mordenite catalysts composited with an alumina salt providing a silica/alumina mole ratio of about 10 to about 30 in the disproportionation of toluene. The reaction conditions proposed in this patent appear similar to those set forth in the aforementioned Mitsche patent and, like the former patent, **Mitsche et al.**, discloses incorporating Group VIB and Group VIII metals into the catalyst.

U.S. Patent No. 4,151,120 to **Marcilly** discloses a process for the manufacture of a hydrocarbon conversion catalyst involving incorporating cobalt, nickel, silver or palladium in a mordenite catalyst having a silica/alumina mole ratio within the range of 10-100. Following incorporation of the metal into the mordenite, the catalyst is dried and subjected to a dry calcination procedure at a temperature within the range of 300-700°C in the presence of an inert or oxidizing gas having a moisture content of less than 1 percent. **Marcilly** discloses various examples of the dismutation of toluene under reaction conditions 420°C, 30·10⁵ Pa (30 bars), a space velocity (WHSV) of 5 and a hydrogen/hydrocarbon mole ratio of 5.

U.S. Patent No. 4,723,048 to **Dufresne et al.** discloses a process for the dismutation of toluene employing a zeolite catalyst modified by the inclusion of metals. The catalyst is described as a sodium containing mordenite in the nature of so-called "wide pore" mordenite, i.e., mordenite with main pores exhibiting a diameter of 0.7-1 nm (7-10 Angstroms) or "small pore" mordenite, mordenites with main pores exhibiting a diameter of 0.4-0.6 nm (4-6 Angstroms). The mordenites are treated to extract sodium therefrom to provide not more than 1 percent by weight sodium ions and preferably not more than 0.5 percent by weight sodium ions. **Dufresne** discloses mordenites having silica/alumina ratios of 10.6 (catalyst A), 14.6 (catalyst B), 25.2 (catalyst C), and 58.6 (catalyst D) modified by the inclusion of nickel ranging from 0.43 weight percent to 2.11 weight percent and by the inclusion of nickel with certain other metals. **Dufresne** discloses activities of the nickel modified catalysts before and after an accelerated aging procedure at conversion rates of 10 percent and 45 percent. Catalyst C containing 1.1 percent nickel showed the best activity with catalyst B containing 2.11 percent nickel and catalyst D having 0.43 percent nickel showing slightly lower activities. The poorest activity was with respect to catalyst A having a nickel content of 1.81 percent.

US 4,956,511 to Butler et al. discloses a process for the disproportionation of toluene over a nickel modified mordenite catalyst under relatively low temperature conditions. A preflush gas such as hydrogen is passed into the reaction zone while withdrawing the gas from the reaction zone and progressively increasing the temperature of the reaction zone to a desired level. A toluene feedstok is then passed into the reaction zone. Hydrogen is supplied with the toluene to the reaction zone, normally at a hydrogen/toluene mole ratio of 4 or less.

The representative prior art discussed above reveals that much of the experimental and investigative work conducted in regard to alkylaromatic disproportionation employing metal promoted mordenite catalysts has been directed toward either achieving a higher level of alkylaromatic conversion or extending catalyst life (ie: controlling catalytic deactivation).

As distinguished from the extant art, the present invention is directed to a start-up procedure for use in the disproportionation of alkylaromatics which results in greater selectivity to desirable benzene product from the very inception of the reaction with a simultaneous and appreciable decrease in by-production of undesirable compounds (hereinafter non-aromatic compounds) due to hydrogenation of the aromatic feed material.

Practical benefits of the instant invention are essentially twofold. Specifically, as an aromatic solvent of comparatively high commercial value and virtually continuous demand, there exists sound economic rationale for increasing the efficiency of the means by which benzene is produced. In addition, and with respect to refining operation, the distillation separation of benzene from undesirable non-aromatic compounds of comparable boiling point range is markedly enhanced due to the significant decrease in non-aromatic by-production, which is the object of the instant invention.

As indicated, common, albeit undesirable, by-products of aromatic hydrogenation (which results when hydrogen is co-fed with an alkylaromatic under disproportionation conditions), including toluene disproportionation, are principally non-aromatic compounds such as methylcyclohexane and, to a lesser extent, other paraffinic and cyclic compounds. Formation of these compounds is particularly observed in association with the presence of a metal promoted mordenite catalyst and, additionally, low reaction temperature. The level of non-aromatic by-production, however, is known to decrease in direct relation to catalyst age. Accordingly, the selectivity to benzene and xylene(s) in toluene disproportionation is dependent upon the by-production level of non-aromatic compounds which, until stabilized, prevent optimum selectivity to the aforesaid desirable products. Applicants have discovered that by controlling the amount of hydrogen cofeed during reaction start-up, the level of non-aromatic production can be limited thereby resulting in greater reaction selectivity to benzene product.

Because it is believed to enhance reaction stability and catalyst aging character, it is generally known that it is desirable to employ a hydrogen cofeed in the disproportionation of alkylaromatics. As disclosed by **Marcilly** (U.S. 4,151,120), **Butler, et. al.** (U.S. 4,665,258) and **Bhavikatti** (Ind. Eng. Chem. Prod. Res. Dev. 1981, 20, 102-105), for example, the prior art generally teaches a hydrogen to toluene molar ratio greater than 3. Applicants' invention, to the contrary, teaches a hydrogen to toluene molar ratio below 3.0 and, preferably, about 1.0 to 2.0.

In view of the above, there exists, accordingly, a need in the art for a start-up process of disproportionating alkylaromatic feed-streams which yields an appreciably desirable and stable concentration of benzene and simultaneous reduction in the by-production of undesirable non-aromatic compounds.

### SUMMARY OF THE INVENTION

In accordance with the present invention, there is provided a process for the disproportionation of a substantially pure toluene feedstock over a metal promoted mordenite catalyst in which the yield of desirable benzene product is enhanced during start-up of the process concurrently with suppression of undesirable non-aromatic product(s), by employing a comparatively low concentration of hydrogen cofeed. In practicing the invention, the toluene feedstock is supplied to the reaction zone containing a metal promoted mordenite catalyst exhibiting a silica to alumina molar ratio of 16:1 to 22:1 and containing an amount of nickel within the range 1.0 - 1.5 weight percent. The reaction zone is operated at a starting temperature of 250°C which is subsequently increased incrementally to within the range of 320° to 330°C and at a pressure of at least 37.92 ·10⁵ Pa (550 psig). The feedstock is supplied to the reaction zone at a rate sufficient to provide a toluene LHSV of 2. Hydrogen gas is co-fed at a rate sufficient to achieve a hydrogen to toluene molar ratio of between 0.5 and 2.5 and, most preferably, 1.0 to 2.0. Following the disproportionation of the toluene to benzene and xylene, the disproportionation product is withdrawn from the reaction zone.

In a further aspect of the invention, it is possible to increase the concentration of hydrogen cofeed and hence the molar ratio of hydrogen to toluene to a value between 3 and 4 at such time (i.e. after 5-10 days) in the reaction that a desirably low and stable concentration of non-aromatic compounds is observed in the product stream. This post start-up increase in hydrogen concentration following stabilized production of non-aromatics is generally believed to enhance the aging character of the catalyst.

### BRIEF DESCRIPTION OF THE DRAWINGS

Figure 1 is a graph illustrating product selectivity to non-aromatic compounds as a function of catalyst age in conjunction with a toluene disproportionation process carried out over a nickel-modified mordenite catalyst wherein hydrogen is co-fed with toluene in molar relationships of 0.5, 1.7 and 3.3.

Figure 2 is a graph illustrating product selectivity to benzene as a function of catalyst age in conjunction with a toluene disproportionation process carried out over a nickel-modified mordenite catalyst wherein hydrogen is co-fed with toluene in molar relationships of 0.5, 1.7 and 3.3.

### DESCRIPTION OF THE PREFERRED EMBODIMENTS

As evidenced in the foregoing discussion, the use of hydrogen in the disproportionation of toluene over a metal modified zeolite catalyst is well known in the art. The present invention, however, provides an improved mode of starting toluene disproportionation over a nickel-modified mordenite catalyst under relatively low temperature conditions providing for desirably low yields of non-aromatic product and resulting in desirably high yields of desirable benzene.

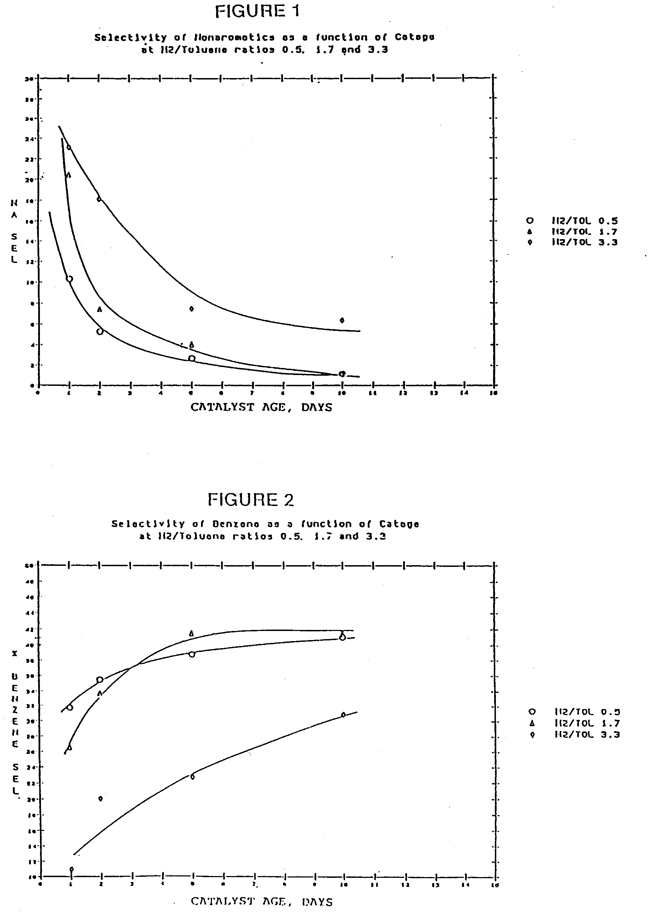

A distinct advantage of the present invention is graphically illustrated in Figure 1 shown herein which indicates that, in utilizing a nickel promoted mordenite catalyst, a toluene conversion level of about 48% can be achieved with minimal formation of non-aromatic compounds in the product stream by reducing the concentration of hydrogen cofeed during reaction start-up. This measure results in a higher yield of desirable benzene product. Although the precise point of optimum hydrogen to toluene ratio was not determined, it was determined that the most significant improvements are achieved between 0.5 - 2.5 and, more preferably, between 1.0 - 2.0 and, further, 1.5.

In accordance with the present invention there is provided a toluene disproportionation process employing a catalyst of the mordenite type modified by the inclusion of a metallic hydrogenation component, more specifically nickel, in which a dramatic decrease of non-aromatic by-product is achieved in conjunction with: toluene conversion of about 48%; relatively low temperature; and a desirably low rate of catalyst deactivation.

The mordenite catalyst employed in the present invention preferably exhibits a silica to alumina molar ratio of between 16:1 and 22:1 and more preferably 18:1. The mordenite catalyst is, additionally, modified by the inclusion of nickel. Applicants' experimentation suggests that the best results are obtained by utilizing a catalyst made up of no less than 1.0 weight per cent nickel. While it is generally known in the art that low nickel content mordenite catalysts are useful in converting toluene and assist in selectivity to benzene and xylenes, they have generally demonstrated poor aging quality (character). Experimentation has demonstrated that, while greater amounts of nickel can be employed without corresponding significant benefit, one preferred nickel content in the catalyst is within the range of 1.2-1.4 weight percent, preferably 1.25 weight percent.

The present invention resulted from a study conducted using three different hydrogen to toluene molar ratios utilizing a laboratory reactor. In each case, the catalyst was pre-dried by heating at a suitable temperature, preferably 250°C, under the flow of hydrogen gas.

Thereafter, a substantially pure toluene feedstock was passed over the reaction zone at a starting temperature of 250°C which was subsequently raised incrementally to within the range of 320°C-330°C, and at a pressure of 41.37 ·10⁵ Pa (600 psig) and feedstock liquid hourly space velocity (LHSV) = 2.0. The hydrogen to toluene molar ratio varied at 0.5, 1.7 and 3.3. Reactor temperature was incrementally increased in an effort to maintain the desired 48% conversion.

## Claims

1. A transalkylation start-up procedure for the process of disproportionating a toluene-containing feedstock over a metal promoted mordenite catalyst to produce benzene and xylene, the steps of said procedure comprising:
(a) passing a cofeed of substantially pure toluene and hydrogen gas into a reaction zone and contacting it with a mordenite catalyst modified by the inclusion of nickel within the reaction zone, said catalyst having a silica to alumina molar ratio in the range of 16:1 to 22:1, said feedstock being supplied to said reaction zone at a rate sufficient to provide a toluene LHSV of 2;
(b) conducting the disproportionation reaction within said reaction zone at a starting reaction temperature of 250°C and a pressure of at least 37.92 ·10⁵ Pa (550 psig);
(c) adjusting said cofeed to obtain a hydrogen to toluene molar ratio within a range of 0.5 and 2.5 at reaction start-up; and
(d) withdrawing disproportionation product containing benzene and xylene from said reaction zone.

2. The process according to claim 1 wherein said reaction temperature is raised incrementally to 320 - 330°C.

3. The process according to claim 1 wherein said toluene disproportionation is around 48% and is maintained by adjusting the reaction temperature.

4. The process according to claim 1 wherein the hydrogen to toluene molar ratio is, more preferably, within the range of 1.0 to 2.0 at reaction start-up.

5. The process according to claim 1 wherein the hydrogen to toluene molar ratio is most preferably 1.5.

6. The process according to claim 1 wherein said hydrogen cofeed concentration is increased after 5 - 10 days of time on stream to result in a hydrogen to toluene molar ratio of between 3 and 4.

7. The process according to claim 1 wherein said mordenite catalyst exhibits a preferable silica to alumina molar ratio of 16:1 to 22:1.

8. The process according to claim 1 wherein said mordenite catalyst exhibits a more preferable silica to alumina molar ratio of 18.

9. The process according to claim 1 wherein the metal promoted mordenite catalyst exhibits a nickel concentration within the range 1.0 - 1.5 weight percent.

10. The process according to claim 1 wherein the metal promoted mordenite catalyst exhibits a nickel concentration within the range 1.2 - 1.4 weight percent.

11. The process according to claim 1 wherein the metal promoted mordenite catalyst exhibits a nickel concentration of about 1.25 weight percent.

## Patentansprüche

1. Transalkylierungs-Anlaufverfahren für ein Verfahren zur Disproportionierung eines toluolhaltigen Speisematerials über einem mit einem Metall aktivierten Mordenitkatalysator zur Herstellung von Benzol und Xylol, wobei das Verfahren die Schritte umfaßt:
(a) daß eine Co-Speisung aus im wesentlichen reinem Toluol und Wasserstoffgas in eine Reaktionszone geführt wird und mit einem Mordenitkatalysator, der durch Einschluß von Nickel modifiziert worden ist, in der Reaktionszone in Kontakt gebracht wird, wobei der Katalysator ein molares Verhältnis von Siliciumdioxid zu Aluminiumoxid im Bereich von 16:1 bis 22:1 aufweist, wobei das Speisematerial mit einer Geschwindigkeit in die Reaktionszone eingespeist wird, die ausreicht, eine Toluol-LHSV von 2 bereitzustellen;
(b) daß die Disproportionierungsreaktion innerhalb der Reaktionszone bei einer Ausgangsreaktionstemperatur von 250 °C und bei einem Druck von mindestens 37,92 · 10⁵ Pa (550 psig) durchgeführt wird;
(c) daß die Co-Speisung so eingestellt wird, daß bei der Anlaufphase der Reaktion ein molares Verhältnis von Wasserstoff zu Toluol im Bereich von 0,5 und 2,5 erhalten wird; und
(d) daß das Benzol und Xylol enthaltende Disproportionierungsprodukt aus der Reaktionszone abgezogen wird.

2. Verfahren nach Anspruch 1, wobei die Reaktionstemperatur schrittweise auf 320 - 330 °C gesteigert wird.

3. Verfahren nach Anspruch 1, wobei die Toluoldisproportionierung etwa 48% beträgt und durch Einstellen der Reaktionstemperatur beibehalten wird.

4. Verfahren nach Anspruch 1, wobei das molare Verhältnis von Wasserstoff zu Toluol bei der Anlaufphase der Reaktion bevorzugter im Bereich von 1,0 bis 2,0 liegt.

5. Verfahren nach Anspruch 1, wobei das molare Verhältnis von Wasserstoff zu Toluol am bevorzugtesten 1,5 beträgt.

6. Verfahren nach Anspruch 1, wobei die Konzentration der Wasserstoff-Co-Speisung nach einer 5 - 10 Tage dauernden Betriebszeit gesteigert wird, was zu einem molaren Verhältnis von Wasserstoff zu Toluol zwischen 3 und 4 führt.

7. Verfahren nach Anspruch 1, wobei der Mordenitkatalysator ein bevorzugtes molares Verhältnis von Siliciumdioxid zu Aluminiumoxid von 16:1 bis 22:1 aufweist.

8. Verfahren nach Anspruch 1, wobei der Mordenitkatalysator ein bevorzugteres molares Verhältnis von Siliciumdioxid zu Aluminiumoxid von 18 aufweist.

9. Verfahren nach Anspruch 1, wobei der mit Metall aktivierte Mordenitkatalysator eine Nickelkonzentration innerhalb des Bereichs von 1,0 - 1,5 Gew.-% aufweist.

10. Verfahren nach Anspruch 1, wobei der mit Metall aktivierte Mordenitkatalysator eine Nickelkonzentration innerhalb des Bereichs von 1,2 - 1,4 Gew.-% aufweist.

11. Verfahren nach Anspruch 1, wobei der mit Metall aktivierte Mordenitkatalysator eine Nickelkonzentration von etwa 1,25 Gew.-% aufweist.

## Revendications

1. Procédure de démarrage de transalkylation dans un procédé de disproportionnement d'une charge contenant du toluène sur un catalyseur mordénite activé d'un métal pour produire du benzène et du xylène, cette procédure comprenant les étapes :
(a) faire passer une charge de toluène et d'hydrogène gazeux substantiellement purs dans une zone de réaction et la mettre en contact dans la zone de réaction avec un catalyseur mordénite modifié par inclusion de nickel, le dit catalyseur ayant un rapport molaire silice:alumine dans la gamme de 16:1 à 22:1, la dite zone de réaction étant approvisionnée par la dite charge à un débit suffisant pour fournir une LHSV du toluène de 2;
(b) mener la réaction de disproportionnement dans la dite zone de réaction à une température de réaction initiale de 250°C et à une pression d'au moins 37,92 .10⁵ Pa (550 psig);
(c) régler la dite charge pour avoir un rapport molaire hydrogène/toluène dans la gamme de 0,5 à 2,5 au démarrage de la réaction; et
(d) retirer de la dite zone de réaction un produit de disproportionnement contenant du benzène et du xylène.

2. Procédé selon la revendication 1 dans lequel la dite température de réaction est augmentée par incrément jusqu'à 320-330°C.

3. Procédé selon la revendication 1 dans lequel le dit disproportionnement de toluène est proche de 48% et est maintenu par réglage de la température de réaction.

4. Procédé selon la revendication 1 dans lequel le rapport molaire hydrogène/toluène est, de préférence, dans la gamme de 1,0 à 2,0 au démarrage de la réaction.

5. Procédé selon la revendication 1 dans lequel le rapport molaire hydrogène/toluène est plus préférentiellement de 1,5.

6. Procédé selon la revendication 1 dans lequel la dite concentration en hydrogène dans la charge est augmentée après 5-10 jours de temps passé en continu, pour arriver à un rapport molaire hydrogène/toluène entre 3 et 4.

7. Procédé selon la revendication 1 dans lequel le dit catalyseur mordénite présente un rapport molaire silice:alumine préféré de 16:1 à 22:1.

8. Procédé selon la revendication 1 dans lequel le dit catalyseur mordénite présente un rapport molaire silice:alumine plus préféré de 18.

9. Procédé selon la revendication 1 dans lequel le dit catalyseur mordénite activé d'un métal présente une concentration en nickel comprise entre 1,0 et 1,5 pourcent en poids.

10. Procédé selon la revendication 1 dans lequel le dit catalyseur mordénite activé d'un métal présente une concentration en nickel comprise entre 1,2 et 1,4 pourcent en poids.

11. Procédé selon la revendication 1 dans lequel le dit catalyseur mordénite activé d'un métal présente une concentration en nickel d'environ 1,25 pourcent en poids.
